# EUROPÄISCHE PATENTSCHRIFT

(11) **EP 0 760 622 B1**
(45) Veröffentlichungstag und Bekanntmachung des Hinweises auf die Patenterteilung: **11.11.1998**
(21) Anmeldenummer: 95922469.2
(22) Anmeldetag: 22.05.1995
(51) Int. Cl.: A61B 5/107, A43D 1/02, G01C 11/06, G01B 11/16, A61F 2/50, G05B 19/42

(54) **VERFAHREN UND ANORDNUNG ZUR DREIDIMENSIONALEN DIGITALISIERTEN ERFASSUNG DER RAUMFORM VON KÖRPERN ODER KÖRPERTEILEN**
DIGITISED SENSING PROCESS AND ARRANGEMENT FOR THE THREE-DIMENSIONAL SHAPE IN SPACE OF BODIES OR BODY PARTS
PROCEDE ET AGENCEMENT DE SAISIE NUMERISEE DE LA FORME TRIDIMENSIONNELLE DE CORPS OU DE PARTIES DE CORPS DANS L'ESPACE

(30) Priorität: 22.05.1994 DE 4417872
(43) Veröffentlichungstag der Anmeldung: 12.03.1997
(73) Patentinhaber: Massen, Robert, Prof. Dr., D-78337 Öhningen (DE)
(72) Erfinder: Massen, Robert, Prof. Dr., D-78337 Öhningen (DE)
(74) Vertreter: Leiser, Gottfried, Dipl.-Ing.
(86) Internationale Anmeldenummer: EP9501934
(87) Internationale Veröffentlichungsnummer: WO9531934

(56) Entgegenhaltungen:
- WO-A-83/04114
- WO-A-90/10194
- WO-A-92/08175
- DE-A- 2 250 679
- GB-A- 2 257 250
- US-A- 4 745 290

## Beschreibung

Die Erfindung betrifft ein Verfahren zur dreidimensionalen digitalisierten Erfassung der Raumform von Körpern oder Körperteilen, insbesondere von menschlichen Körperteilen oder Gliedmaßen zur weitgehend automatischen Herstellung von Prothesen oder angepaßten Formteilen, bei welchem der Körper oder Körperteil zusammen mit einem auf seine Oberfläche aufgebrachten kontrastreichen Muster von mehreren nach Art von Stereokameras mit sich überlappenden Bildbereichen zusammenwirkenden Kameras aufgenommen und durch automatische Zuordnung der sich in den aufgenommenen Bildern jeweils entsprechenden Mustern mittels einer Recheneinheit die digitalisierten Daten der Raumform gewonnen werden.

Ein Verfahren dieser Art ist aus der DD-Patentschrift 78 310 zum Zweck der Erfassung anthropometrischer Daten bekannt. Bei diesem verfahren werden unter Verwendung einer Stereomeßkamera von einer Versuchsperson nach Einnahme einer festgelegten Haltung und ihrer Fixierung durch eine geeignete Vorrichtung von einer Körperseite eine oder mehrere Aufnahmen hergestellt, wobei die Körperoberfläche vorher durch eine Anordnung von Strukturwänden und Lichtquellen mit einer Schatten- oder Rasterstruktur versehen wurde. Die Stereoaufnahmen werden mittels Stereoautographen oder Stereokomparatoren ausgewertet, und die aus den Bildern gewonnenen Daten werden durch elektronische Rechenautomaten ausgewertet und weiterverarbeitet.

Dieses Verfahren ist zur dreidimensionalen Erfassung der Raumform von Körpern oder Körperteilen für eine weitgehend automatische Herstellung von Prothesen oder angepaßten Formteilen nicht gut geeignet. Für diesen Zweck muß nämlich der Körper oder Körperteil von verschiedenen Seiten in möglichst kurzer Zeit, vorzugsweise gleichzeitig, aufgenommen werden, da sich der Patient während der optischen Vermessung nicht bewegen darf. Der Erfüllung dieser Forderung steht insbesondere die strukturierte Beleuchtung mittels Strukturwänden und Lichtquellen entgegen, die für die verschiedenen Aufnahmerichtungen nicht gleichzeitig erfolgen kann. Da die optische Erfassung auch unabhängig vom Hauttyp und eventueller Behaarung erfolgen muß, sind aufwendige und zeitraubende Vorbereitungsmaßnahmen erforderlich, wie das Auftragen von gut reflektierender Schminke oder sogar das Enthaaren durch Rasieren.

Ferner bestehen auch hygienische Probleme. Da Teile der Aufnahmeeinrichtung nacheinander von nackten Körperteilen verschiedener Patienten berührt werden, ist es erforderlich, diese Teile so zu gestalten, daß sie regelmäßig desinfiziert werden können. Diese Desinfektionsfähigkeit bedingt die Verwendung von teuren Materialien, die ebenso wie der für die Desinfektionen benötigte Zeitaufwand die Kosten erhöhen.

Es ist andererseits bekannt, die Raumform von Körperteilen oder Gliedmaßen mittels aktiver Laserscanner zu erfassen. Bei einem aus der PCT-Veröffentlichung WO 92/08175 bekannten Verfahren dieser Art wird mittels einer Laserquelle auf den mit einem dünnen elastischen Überzug überzogenen Körperteil eine einzelne Lichtlinie projiziert, die dem Profil des Körperteils folgt. Durch Triangulation mit Hilfe eines optischen Sensors werden die räumlichen Koordinaten der einzelnen Punkte dieser Profillinie bestimmt. Der Vorgang wird für weitere Profillinien wiederholt, wobei ein den Laserscanner tragender Drehtisch jeweils um einen kleinen Winkel verdreht wird, bis schließlich der Körperteil vollständig abgetastet ist. Es ist bei diesem bekannten System auch vorgesehen, daß kritische Stellen des Körperteils auf dem Überzug durch Markierungen gekennzeichnet werden, die das Laserlicht nicht reflektieren. Diese mit aktiven Laserscannern arbeitenden Systeme sind jedoch im Vergleich zu den mit passiven Stereokameras arbeitenden photogrammetrischen Systemen sehr aufwendig. Ferner besteht das Problem, daß selbst bei schnell arbeitenden Systemen die gesamte dreidimensionale Erfassung eines Körperteils eine Zeitspanne von etwa 10 bis 15 Sekunden dauert, in der das Körperteil nicht bewegt werden darf.

Aufgabe der Erfindung ist demgegenüber die Schaffung eines passiven photogrammetrischen Mehrkameraverfahrens, das mit geringem Aufwand eine sehr schnelle dreidimensionale Erfassung der Raumform eines Körpers oder Körperteils ermöglicht, Vorbehandlungen des aufzunehmenden Körpers oder Körperteils erübrigt und ohne die Notwendigkeit von Desinfektionen die hygienischen Anforderungen erfüllt.

Ausgehend von einem Verfahren der eingangs angegebenen Art wird diese Aufgabe nach der Erfindung dadurch gelöst, daß der Körper oder Körperteil vor der Aufnahme mit einem dünnen, enganliegenden Überzug bedeckt wird, der das kontrastreiche Muster trägt.

Bei dem erfindungsgemäßen Verfahren erfüllt der dünne, enganliegende Überzug mehrere Funktionen:
1. Er trägt das für die photogrammetrische Auswertung erforderliche Muster, das dadurch ohne die Notwendigkeit aufwendiger Projektionen die gesamte aufzunehmende Oberfläche des Körpers oder Körperteils bedeckt. Dadurch ist es insbesondere möglich, die Aufnahmen aus verschiedenen Richtungen und sogar aus allen erforderlichen Richtungen gleichzeitig zu machen, wenn eine entsprechende Anzahl von Kameras vorhanden ist. Der hierfür benötigte Aufwand ist dennoch gering, da als Kameras elektronische Bildsensoren verwendet werden können, die als Massenartikel sehr kostengünstig erhältlich sind. Als Beleuchtung genügt eine einfache und billige diffuse Beleuchtung.
2. Infolge der Verwendung des Überzugs mit dem darauf angebrachten Muster sind unabhängig von der Beschaffenheit des aufzunehmenden Körpers oder Körperteils stets gleichbleibende optische Bedingungen gewährleistet. Daher entfällt die Notwendigkeit von aufwendigen Vorbereitungen, wie Auftragen von Schminke oder Enthaarung.
3. Da der mit dem Überzug bedeckte Körper oder Körperteil nicht direkt mit Teilen der Aufnahmeeinrichtung in Berührung kommen kann, ist stets eine vollständige Hygiene gewährleistet, ohne daß Desinfektionen notwendig sind. Dies gilt insbesondere, wenn der Überzug als billiger Einmalartikel hergestellt und nur einmal verwendet wird.
4. Der Überzug bedeckt auch abstoßende Wunden oder Verstümmelungen von amputierten Körperteilen und Gliedmaßen, wodurch psychische Belastungen sowohl des Patienten als auch des die Aufnahmen durchführenden Personals vermindert werden.

Vorteilhafte Ausgestaltungen und Weiterbildungen des erfindungsgemäßen Verfahrens sind in den Unteransprüchen gekennzeichnet. Die Erfindung betrifft ebenfalls eine Anordnung zur Durchführung des Verfahrens.

Weitere Merkmale und Vorteile der Erfindung ergeben sich aus der folgenden Beschreibung eines Ausführungsbeispiels anhand der Zeichnungen. In den Zeichnungen zeigen:
- Fig. 1: einen mit einem Überzug versehenen Fuß als Beispiel für ein Körperteil, dessen Raumform dreidimensional erfaßt werden soll,
- Fig. 2: verschiedene Beispiele von Mustern, die auf den Überzug aufgebracht werden können,
- Fig. 3: eine schmatische Darstellung der Aufnahmevorrichtung,
- Fig. 4: einen Kalibrationskörper zur Kalibrierung des Systems,
- Fig. 5: ein Blockschema des gesamten Erfassungssystems und
- Fig. 6: das Blockschema einer abgeänderten Ausführungsform des Erfassungssystems.

Die dreidimensionale Erfassung der Raumform eines Körpers oder Körperteils mit mehreren Kameras in einer Stereoanordnung bedingt, daß jeweils mindestens zwei Kameras sich überlappende Bildbereiche erfassen. Gleiche Strukturen in dem gemeinsamen Bildbereich werden wegen der Aufnahme aus unterschiedlichen Aufnahmerichtungen in der jeweiligen Bildebene jeder Kamera an unterschiedlichen Stellen abgebildet. Der Versatz dieser Strukturen zwischen den von jeweils zwei Kameras aufgenommenen Bildern ist proportional zur Entfernung von den beiden Kameras und kann, wenn die Position der Kameras im Raum bekannt ist, in XYZ-Koordinaten umgerechnet werden. Die Stereophotographie kann auch auf mehr als zwei Kameras ausgeweitet werden und erlaubt insbesondere auch dann noch eindeutige Messungen, wenn keine rein zufälligen Strukturen in den gemeinsamen Bildbereichen sichtbar sind. Die mathematisch-photogrammetrischen Grundlagen der Stereophotogrammetrie sowie der Kalibrierung solcher Anordnungen sind dem Fachmann der Photogrammetrie bekannt und brauchen daher nicht weiter erläutert zu werden.

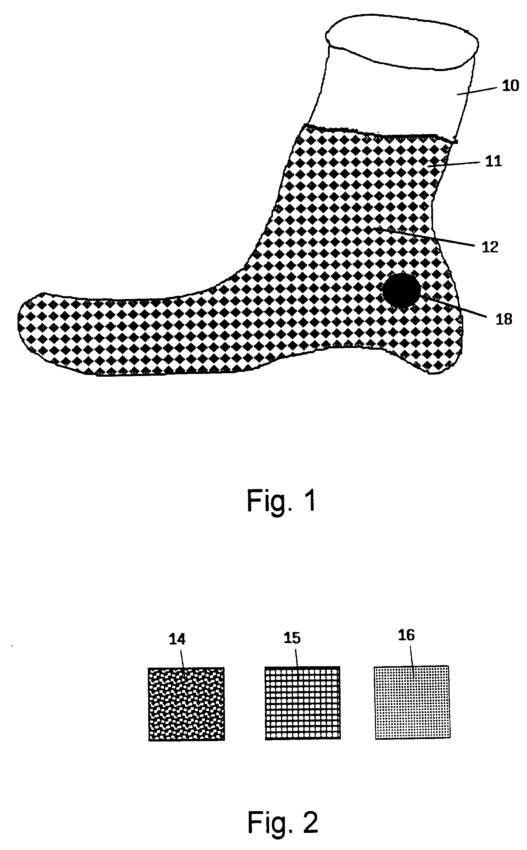

Als Beispiel für einen Körperteil, dessen Raumform dreidimensional erfaßt werden soll, ist in Fig. 1 ein Fuß 10 dargestellt. Der Fuß 10 ist mit einem dünnen, enganliegenden, vorzugsweise elastischen Überzug 11 überzogen, der aus hygienischen Gründen nur einmal verwendet wird und im Falle des Fußes 10 die Form eines Strumpfes hat. Durch die Verwendung des Überzugs 11 entfallen sämtliche Schmink- und Enthaarprozeduren, welche sonst erforderlich sind, wenn dunkle oder stark behaarte Hautpartien optisch dreidimensional erfaßt werden sollen. Außerdem wird durch die Verwendung des hygienischen Einmalstrumpfes verhindert, daß nackte Körperteile mit Halterungen der Aufnahmevorrichtung in Berührung kommen und diese vor jeder Aufnahme desinfiziert werden müssen. Da zahlreiche Patienten mit kranken Füssen auch an Kauterkrankungen wie Ekzemen, entzündlichen Druckstellen usw. leiden, ist eine hygienische Abschirmung besonders wichtig.

Der Überzug 11 besteht vorzugsweise aus einem feinmaschig gewebten oder gewirkten elastischen Textilmaterial, beispielsweise einem sogenannten Stretchgewebe. Er kann jedoch auch aus einer dünnen Folie aus Gummi oder einem elastomeren Kunststoff hergestellt werden. Ferner kann der Überzug durch eine Schrumpffolie gebildet werden, die sich durch Erhitzen mit warmer Luft oder durch Aufsprühen eines chemischen Reagenz eng um den Körper oder Körperteil zusammenzieht. Schließlich ist es auch möglich, den Überzug durch Aufsprühen eines Beschichtungsmaterials unmittelbar auf dem Körper oder Körperteil zu bilden.

Die Oberfläche des Überzugs 11 ist hell und mit einem kontrastreichen, für die Stereoauswertung geeigneten Muster 12 versehen. Dieses Muster kann eine zufällige, unregelmäßige Textur darstellen, welche bei der Stereoauswertung ein eindeutiges korrelatives Matching erlaubt, oder es kann, wie in Fig. 1 dargestellt, ein regelmäßiges und periodisches Muster sein, welches durch Auswertung von mehr als zwei Kamerabildern, die einen gemeinsamen Überlappungsbereich aufzeigen, trotz der periodischen Struktur zu eindeutigen Oberflächenkoordinaten führt. Solche geeigneten Muster sind dem Fachmann der Photogrammetrie bekannt. In Fig. 2 sind als Beispiele ein unregelmäßiges Muster 14, ein gitterförmiges Muster 15 und ein Punktmuster 16 dargestellt, die alle für diesen Zweck geeignet sind. Das Muster 12 kann auf das Material des Überzugs 11 aufgedruckt sein oder auf eine andere, dem Fachmann bekannte Weise aufgebracht sein, beispielsweise durch Aufsprühen. Im Falle eines gewebten oder gewirkten Überzugs kann das Muster auch eingewebt bzw. eingewirkt sein.

Auf dem mit dem Muster 12 versehenen Überzug 11 können zusätzlich bestimmte markante Stellen des Fußes mit Hilfe eines Stiftes markiert werden, z.B. die Position des Knöchels durch eine Markierung 18, damit bei der späteren Auswertung der aufgenommenen Bilder eine Zuordnung von Oberflächenkoordinaten zu signifikanten Punkten, Linien oder Regionen des Fußes möglich ist. Solche Markierungen können leicht mit bekannten Verfahren der Bildverarbeitung aus den Kamerabilder erkannt und von den für die Stereoauswertung bestimmten Mustern unterschieden werden. Selbstverständlich lassen sich auch farbige Markierungen und Muster verwenden, wenn die eingesetzten Kameras farbtüchtig sind.

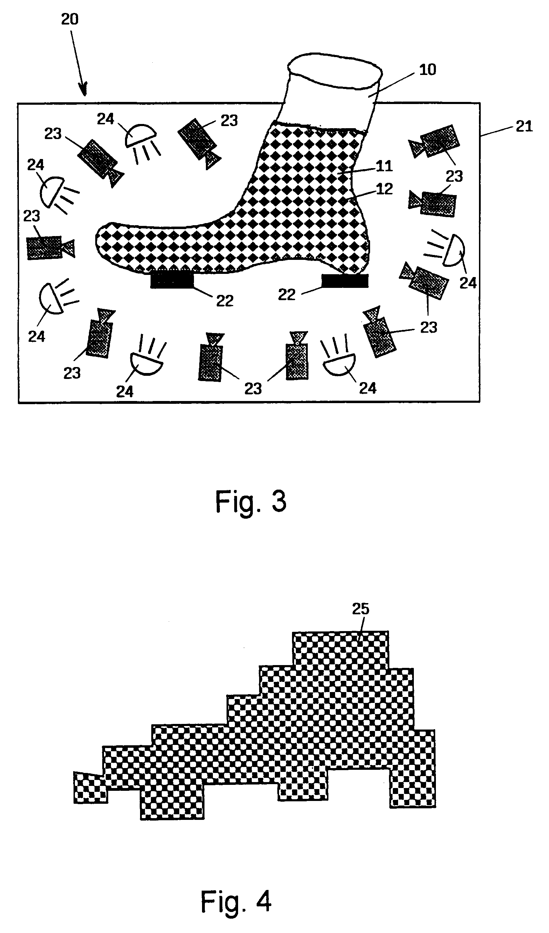

In Fig. 3 ist schematisch dargestellt, wie die dreidimensionale Erfassung der Raumform des Fußes 10 in einer Aufnahmevorrichtung 20 erfolgt. Die Aufnahmevorrichtung 20 hat ein kastenförmiges Gehäuse 21, in das der Fuß 10 von oben eingeführt wird. Auflager 22 halten den Fuß 10 in einer vorbestimmten Lage fest. Im Innern des Gehäuses 21 sind mehrere Kameras 23 so um den Fuß 10 herum angebracht, daß sie den gesamten Fuß aus allen Richtungen vollständig aufnehmen können und daß sich die Bildbereiche paarweise oder gruppenweise derart teilweise überlappen, daß jeweils wenigstens zwei Kameras einen gemeinsamen Bildbereich erfassen. Ferner sind in dem Gehäuse 21 Lampen 24 angebracht, die den gesamten Fuß diffus beleuchten. Anstelle der in Fig. 3 dargestellten Vielzahl von punktförmigen Lichtquellen können auch einige langgestreckte Lichtquellen, wie Leuchtstoffröhren, zur diffusen Beleuchtung des Fußes verwendet werden. In jedem Fall ist die Beleuchtungseinrichtung einfach und billig; infolge der Verwendung des auf den Überzug 11 aufgebrachten Musters 12 entfällt die Notwendigkeit einer kostenintensiven strukturierten Beleuchtung.

Die Kameras 23 sind elektronische Bildsensoren, die die aufgenommenen Bilder in elektrische Bildsignale umsetzen. Hierfür werden vorzugsweise Halbleiterkameras verwendet, die als Massenartikel sehr kostengünstig erhältlich sind, so daß auch die Verwendung von zahlreichen Kameras immer noch eine wirtschaftliche Lösung darstellt. Insbesondere die Entwicklung von 1-Chip-Kameras erlaubt den Einsatz auch einer großen Zahl von Kameras bei niedrigen Systemkosten. Als besonders kostengünstig wirkt sich die Tatsache aus, daß in der Aufnahmevorrichtung keine bewegten Teile, wie Drehtische oder dgl., benötigt werden.

Vorzugsweise erfolgt die Aufnahme des gesamten Fußes durch alle Kameras gleichzeitig, so daß nur eine sehr kurze Aufnahmezeit erforderlich ist und damit die Zeit der Unbeweglichkeit für den Patienten angenehm kurz ist.

Zur Kalibrierung des Systems wird ein Kalibrationskörper 25, wie er schematisch in Fig. 4 dargestellt ist, in einer Paßstellung in der Aufnahmevorrichtung 20 von allen Kameras 23 aufgenommen und vermessen. Der Kalibrationskörper 25 hat eine bekannte Geometrie und trägt auf seiner Oberfläche ein Muster nach Art des auf den Überzug 11 aufgebrachten Musters 12. Dadurch, daß alle Ebenen, Markierungen und Positionen bekannt sind, können alle gemessenen Koordinaten in ein Maschinenkoordinatensystem überführt werden, welches durch die Paßstellung des Kalibrationskörpers gegeben ist. Die Umrechnung in ein objektbezogenes Koordinatensystem, welches z.B. durch auf den Überzug aufgebrachte Markierungen definiert ist, ist damit jederzeit möglich.

Die klassische Photogrammetrie kennt auch Methoden zur genauen dreidimensionalen Vermessung mit Hilfe mehrer Kameras, deren Positionen im Raum nicht bekannt sind, welche aber jeweils eine gewisse Anzahl gleicher Marken erfassen. Auch diese Verfahren können zur Anwendung kommen, wobei die kontrastreichen Muster 12 als Marken verwendet werden.

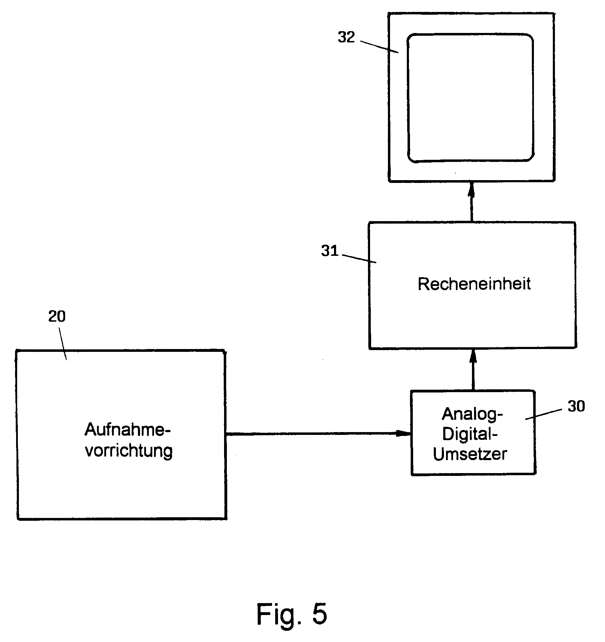

Fig. 5 zeigt den gesamten Aufbau des Erfassungssystems. Die von den Kameras 23 in der Aufnahmevorrichtung 20 gelieferten Bildsignale werden, falls sie nicht bereits in digitaler Form vorliegen, in einem Analog-Digital-Umsetzer 30 digitalisiert und einer Recheneinheit 31 zugeführt, die mit einem Monitor 32 verbunden ist, der eine visuelle Kontrolle der einzelnen Bilder erlaubt. Die Recheneinheit 31 führt die photogrammetrischen Berechnungen aus und erzeugt einen 3D-Datensatz, welcher ebenfalls zur Kontrolle auf dem Monitor 32 angezeigt werden kann.

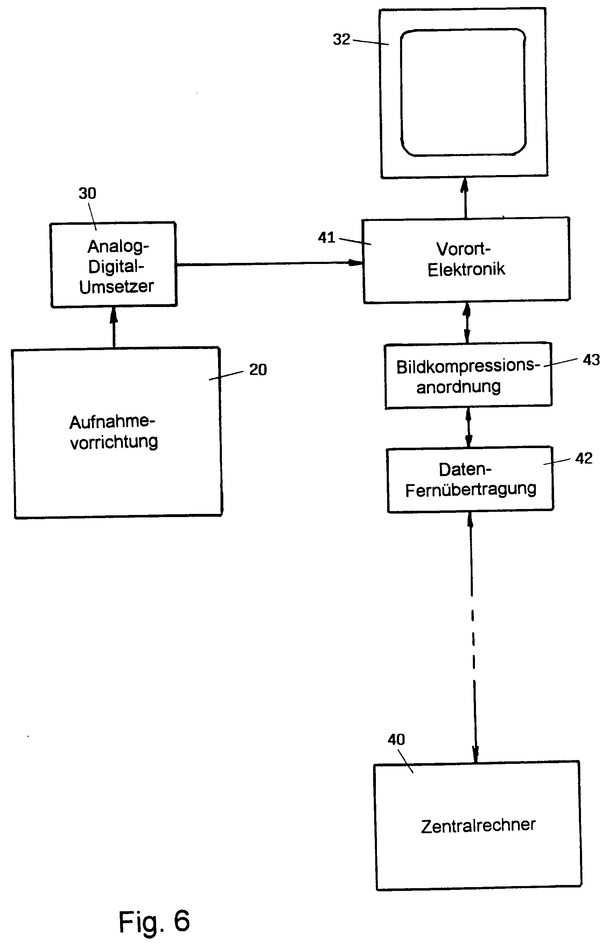

Fig. 6 zeigt eine abgeänderte Ausführungsform des Erfassungssystems, bei der aus Kostengründen die photogrammetrische Berechnung auf einen Zentralrechner 40 ausgelagert wird. Die am Erfassungsort befindliche Vorort-Elektronik 41 dient dann nur zur Wiedergabe der einzelnen Kamerabilder auf dem Monitor 32 und zur Übertragung der erzeugten Rohbilddaten zu dem Zentralrechner 40 mittels eines geeigneten Datenfernübertragungssystems 42. Gegebenenfalls können die Bildsignale vor der Übertragung in einer Bildkompressionsanordnung 43 komprimiert werden. Die vom Zentralrechner 40 ausgewerteten Bildsignale können zur Kontrolle zur Vorortelektronik 41 zurückübertragen und als Bilder auf dem Monitor 32 wiedergegeben werden.

In jedem Fall können die von der Recheneinheit 31 bzw. vom Zentralrechner 40 berechneten 3D-Daten zur automatischen Fertigung einer Schuhleiste, eines Fußbettes oder einer Fußform verwendet werden.

Die gezeigte Vermessung von Füssen ist nur ein Anwendungsbeispiel. Auch bei der Vermessung anderer Gliedmaßen und Körperteile treten die besprochenen technischen, hygienischen und wirtschaftlichen Probleme auf, welche sich durch die Verwendung eines hygienischen, flexiblen Einmalüberzugs beheben lassen, der eine helle Oberfläche mit dazu kontrastierender Musterung aufweist und damit die passive optische Erfassung der Raumform mit einer Mehrkamera-Anordnung schnell und kostengünstig erlaubt. Weitere Beispiele sind z.B. die Vermessung von Gesäßen zur Herstellung angepaßter Sitzschalen, die Vermessung von Rückenpartien zur Herstellung angepaßter Sitzlehnen, die Vermessung von Arm- und Beinstümpfen bei der Herstellung von Gliedprothesen usw.. Der Anwendungsbereich geht damit auch über die rein medizinischen Anwendungen hinaus und trifft immer dann zu, wenn individuell an eine Körperform angepaßte Formen erstellt werden sollen.

Das beschriebene Verfahren läßt sich selbstverständlich auch auf die digitalisierte Erfassung der Raumform von Objekten übertragen, welche keine menschlichen Körperteile sind, wie Designmodelle, Entwurfstudien oder dgl.

## Patentansprüche

1. Verfahren zur dreidimensionalen digitalisierten Erfassung der Raumforn von Körpern oder Körperteilen (10), insbesondere von menschlichen Körperteilen oder Gliedmaßen zur weitgehend automatischen Herstellung von Prothesen oder angepaßten Formteilen, bei welchem der Körper oder Körperteil (10) zusammen mit einem auf seine Oberfläche aufgebrachten kontrastreichen Muster (12) von mehreren nach Art von Stereokameras mit sich überlappenden Bildbereichen zusammenwirkenden Kameras (23) aufgenommen wird und durch automatische Zuordnung der sich in den aufgenommenen Bildern jeweils entsprechenden Mustern (12) mittels einer Recheneinheit (31; 40) die digitalisierten Daten der Raumform gewonnen werden, dadurch gekennzeichnet, daß die aufzunehmende Oberfläche des Körpers oder Körperteils (10) vor der Aufnahme mit einem dünnen, enganliegenden Überzug (11) bedeckt wird, der das kontrastreiche Muster (12) trägt.

2. Verfahren nach Anspruch 1, dadurch gekennzeichnet, daß der Überzug (11) eine helle Oberfläche hat, auf die das kontrastreiche Muster (12) in dunklerer Farbe aufgebracht ist

3. Verfahren nach Anspruch 1 oder 2, dadurch gekennzeichnet, daß das kontrastreiche Muster (14) zufällig ist.

4. Verfahren nach Anspruch 1 oder 2, dadurch gekennzeichnet, daß das kontrastreiche Muster (12; 15; 16) periodisch ist.

5. Verfahren nach einem der Ansprüche 1 bis 4, dadurch gekennzeichnet, daß der Überzug (11) elastisch ist.

6. Verfahren nach Anspruch 5, dadurch gekennzeichnet, daß der Überzug (11) aus Gummi oder einem elastomeren Kunststoff besteht.

7. Verfahren nach einem der Ansprüche 1 bis 5, dadurch gekennzeichnet, daß der Überzug (11) aus einem gewebten oder gewirkten Textilmaterial besteht.

8. Verfahren nach Anspruch 7, dadurch gekennzeichnet, daß das Muster (12) in den Überzug (11) eingewebt bzw. eingewirkt ist.

9. Verfahren nach einem der Ansprüche 1 bis 7, dadurch gekennzeichnet, daß das Muster (12) auf den Überzug (11) aufgedruckt ist.

10. Verfahren nach einem der vorhergehenden Ansprüche, dadurch gekennzeichnet, daß nach dem Anbringen des Überzugs (11) und vor der Aufnahme auf dem mit dem kontrastreichen Muster (12) versehenen Überzug (11) bestimmte Stellen des Körpers oder Körperteils (10) von Hand mit Markierungen (18) versehen werden und diese Markierungen (18) in den aufgenommenen Bildern mit Verfahren der Bildverarbeitung erkannt und als Hilfen für die Zuordnung von dreidimensionalen Oberflächenkoordinaten zu den markierten Stellen des Körpers oder Körperteils (10) verwendet werden.

11. Verfahren nach einem der vorhergehenden Ansprüche, dadurch gekennzeichnet, daß die verwendeten Kameras (23) elektronische Bildsensoren sind, die elektrische Bildsignale abgeben, die die aufgenommenen Bilder darstellen, und daß die von den Bildsensoren (23) gelieferten Bildsignale digitalisiert (in 30) und an die Recheneinheit (31; 40) weitergeleitet werden, in der nach bekannten Verfahren der Photogrammetrie die dreidimensionalen Koordinaten der Oberfläche des Körpers oder Körperteils (10) berechnet werden.

12. Verfahren nach Anspruch 11, dadurch gekennzeichnet, daß die Bildsignale direkt oder nach einer Datenkompression (in 43) durch Datenfernübertragung (42) zu einer zentralen Recheinheit (40) zur photogrammetrischen Auswertung übertragen werden.

13. Verfahren nach Anspruch 12, dadurch gekennzeichnet, daß zur Kontrolle der korrekten Digitalisierung und Auswertung die digitalisierten und ausgewerteten Bildsignale von der zentralen Recheneinheit (40) zur Aufnahmestelle zurückübertragen und dort (in 32) angezeigt werden.

14. Verfahren nach einem der vorhergehenden Ansprüche, dadurch gekennzeichnet, daß für die Aufnahme menschlicher Körperteile oder Gliedmaße (10) die Überzüge (11) aus hygienischen Gründen als nur einmal zu verwendende Artikel ausgelegt und eingesetzt werden.

15. Anordnung zur Durchführung des Verfahrens gemäß einem der Ansprüche 1 bis 14, bestehend aus einem dünnen, ein kontrastreiches Muster (12) tragenden Überzug (11) zur enganliegenden Bedeckung eines Körpers oder Körperteils (10) in Kombination mit einer Aufnahmevorrichtung (20) zur Aufnahme des mit dem Überzug (11) bedeckten Körpers oder Körperteils (10), die eine Beleuchtungseinrichtung (24) zur diffusen Beleuchtung des Körpers oder Körperteils (10) sowie mehrere elektronische Bildsensoren (23) mit sich paarweise oder gruppenweise überlappenden Bildbereichen enthält, und einer Recheneinheit (31; 40), zu der die Bildsignale aller Bildsensoren (23) übertragen werden.

16. Anordnung nach Anspruch 15, dadurch gekennzeichnet, daß die von dem dünnen, enganliegenden elastischen Überzug (11) getragenen Muster (12) und/oder Markierungen (18) farblich kontrastieren und daß die elektronischen Bildsensoren (23) farbtüchtige Bildsensoren sind.

17. Anordnung nach Anspruch 15 oder 16, dadurch gekennzeichnet, daß die Bildsensoren (23) in der Aufnahmevorrichtung (20) so angeordnet sind, daß sie die gesamte aufzunehmende Oberfläche des Körpers oder Körperteils (10) erfassen, und daß die Aufnahme durch alle Bildsensoren (23) gleichzeitig erfolgt.

## Claims

1. A method for digitized three-dimensional sensing of the shape of bodies or body parts (10), particularly human body parts or limbs, for largely automated production of artificial limbs or adapted body parts, in which the body or body part (10) together with a high-contrast pattern (12) on the surface thereof is imaged by several cameras (23) cooperating in the manner of stereo cameras having overlapping image areas and in which the digitized data of the three-dimensional shape are obtained by automatic association of corresponding patterns (12) recorded in the images by means of a computer (31; 40), characterized in that the surface of the body or body part (10) to be imaged is covered prior to imaging with a thin, tight-fitting envelope (11) carrying the high-contrast pattern (12).

2. The method as set forth in claim 1, characterized in that the envelope (11) has a light surface on which the high-contrast pattern (12) is applied in a darker colour.

3. The method as set forth in claim 1 or 2, characterized in that the high-contrast pattern (14) is random.

4. The method as set forth in claim 1 or 2, characterized in that the high-contrast pattern (12; 15; 16) is periodic.

5. The method as set forth in any one of the claims 1 to 4, characterized in that the envelope (11) is elastic.

6. The method as set forth in claim 5, characterized in that the envelope (11) consists of rubber or an elastomer plastics material.

7. The method as set forth in any one of the claims 1 to 5, characterized in that the envelope (11) consists of a woven or knitted textile material.

8. The method as set forth in claim 7, characterized in that the pattern (12) is woven or knitted in the envelope (11).

9. The method as set forth in any one of the claims 1 to 7, characterized in that the pattern (12) is printed on the envelope (11).

10. The method as set forth in any one of the preceding claims, characterized in that after application of the envelope (11) and prior to imaging, certain positions of the body or body part (10) are provided manually with markings (18) on the envelope (11) provided with the high-contrast pattern (12), and these markings (18) are recognized in the recorded images by image processing methods and employed as assistance in assigning three-dimensional surface coordinates to the marked positions of the body or body part (10).

11. The method as set forth in any one of the preceding claims, characterized in that the cameras (23) used are electronic image sensors, outputting electrical image signals representing the recorded images, and that the image signals furnished by the image sensors (23) are digitized (in 30) and passed on to the computer (31; 40) in which according to known photogrammetric methods the three-dimensional coordinates of the surface of the body or body part (10) are computed.

12. The method as set forth in claim 11, characterized in that the image signals are transmitted directly or after data compression (in 43) by a data link (42) to a host computer (40) for photogrammetric evaluation.

13. The method set forth in claim 12, characterized in that for checking for correct digitization and evaluation the digitized and evaluated image signals are transferred back from the host computer (40) to the imaging location for display there (in 32).

14. The method set forth in any one of the preceding claims, characterized in that for imaging human body parts or limbs (10) the envelopes (11) are designed and employed for reasons of hygiene as disposable articles.

15. An arrangement for implementing the method as set forth in any one of the claims 1 to 14, comprising a thin envelope (11) bearing a high-contrast pattern (12) for a tight-fitting covering of a body or body part (10), in combination with an imaging device (20) for imaging the body or body part (10) covered by the envelope (10), containing illumination means (24) for diffused illumination of the body or body part (10), as well as several electronic image sensors (23) having image areas overlapping in pairs or group-wise, and a computer (31; 40) to which the image signals of all image sensors (23) are transferred.

16. The arrangement as set forth in claim 15, characterized in that the patterns (12) and/or markings (18) carried by the thin, tight-fitting elastic envelope (11) contrast in colour and said electronic image sensors (23) are colour-sensitive image sensors.

17. The arrangement as set forth in claim 15 or 16, characterized in that the image sensors (23) in the imaging device (20) are arranged so that they sense the complete surface of the body or body part (10) to be imaged and that imaging is done by all image sensors (23) simultaneously.

## Revendications

1. Procédé pour la saisie numérisée en trois dimensions de la forme volumétrique de corps ou de parties (10) du corps, notamment de parties ou de membres du corps humain, pour la fabrication largement automatique de prothèses ou de pièces de forme, procédé dans lequel le corps ou la partie (10) du corps, en association avec un dessin contrasté (12), appliqué sur sa surface, est filmé par plusieurs caméras (23), à la façon de caméras stéréo, fonctionnant ensemble en formant des zones d*'*images se chevauchant, et procédé dans lequel on obtient les données numérisées de la forme volumétrique par l*'*affectation automatique, au moyen d*'*une unité de calcul (31 ; 40), des dessins (12) se correspondant respectivement dans les images filmées, caractérisé en ce que la surface du corps ou de la partie (10) du corps à filmer, avant la prise de vues, est recouverte d'un mince revêtement (11), lequel revêtement porte le dessin contrasté (12).

2. Procédé selon la revendication 1, caractérisé en ce que le revêtement (11) comprend une surface claire sur laquelle le dessin contrasté (12) est appliqué en couleur foncée.

3. Procédé selon la revendication 1 ou 2, caractérisé en ce que le dessin contrasté (14) est aléatoire.

4. Procédé selon la revendication 1 ou 2, caractérisé en ce que le dessin contrasté (12 ; 15 ; 16) est périodique.

5. Procédé selon l*'*une quelconque des revendications 1 à 4, caractérisé en ce que le revêtement (11) est élastique.

6. Procédé selon la revendication 5, caractérisé en ce que le revêtement (11) est en caoutchouc ou dans une matière plastique à base d'élastomère.

7. Procédé selon l'une quelconque des revendications 1 à 5, caractérisé en ce que le revêtement (11) se compose d'une matière textile tissée ou tricotée.

8. Procédé selon la revendication 7, caractérisé en ce que le dessin (12) est tissé ou tricoté dans le revêtement.

9. Procédé selon l'une quelconque des revendications 1 à 7, caractérisé en ce que le dessin (12) est imprimé sur le revêtement (11).

10. Procédé selon l'une quelconque des revendications précédentes, caractérisé en ce que, après l'application du revêtement (11) et avant la prise de vues, des marques (18) sont effectuées à la main en différents endroits du corps ou de la partie (10) du corps, lesdites marques étant effectuées sur le revêtement (11) doté du dessin contrasté (12), et en ce que ces marques (18), grâce au procédé du traitement d'images, sont identifiées dans les images filmées, et en ce que lesdites marques sont utilisées comme aides pour l'affectation de coordonnées de surface tridimensionnelles, aux endroits marqués du corps ou de la partie (10) du corps.

11. Procédé selon l'une quelconque des revendications précédentes, caractérisé en ce que les caméras utilisées (23) sont des capteurs électroniques d'images qui fournissent des signaux électriques d'images qui représentent les images filmées, et en ce que les signaux d'images fournis par les capteurs d'images (23) sont numérisés (en 30) et transmis à l'unité de calcul (31 ; 40) où, suivant des procédés connus de la photogrammétrie, on calcule les coordonnées tridimensionnelles de la surface du corps ou de la partie (10) du corps.

12. Procédé selon la revendication 11, caractérisé en ce que les signaux d'images sont transmis par télétransmission de données (42), directement ou après une compression de données (en 43), à une unité centrale de calcul (40), pour effectuer l'analyse photogrammétrique.

13. Procédé selon la revendication 12, caractérisé en ce que, pour le contrôle de la numérisation et de l'analyse correctes, les signaux d'images numérisés et analysés sont retransmis, de l'unité centrale de calcul (40) jusqu'à l'emplacement de la prise de vues, où lesdits signaux sont visualisés (en 32).

14. Procédé selon l'une quelconque des revendications précédentes, caractérisé en ce que, pour la prise de vues de parties ou de membres du corps humain (10), les revêtements (11), pour des raisons d'hygiène, sont conçus et appliqués comme des articles à n'utiliser qu'une seule fois.

15. Agencement pour l'exécution du procédé selon l'une quelconque des revendications 1 à 14, se composant d'un revêtement mince (11), portant un dessin contrasté (12), pour revêtir, de façon bien ajustée, un corps ou la partie (10) d'un corps, en combinaison avec un dispositif (20) de prise de vues pour la prise de vues du corps ou de la partie (10) du corps recouvert du revêtement (11), lequel dispositif de prise de vues comprend un dispositif d'éclairage (24) pour l'éclairage diffus du corps ou de la partie (10) du corps, ainsi que plusieurs capteurs électroniques (23) d'images comprenant des zones d'images se chevauchant par paires ou par groupes, et en combinaison avec une unité de calcul (31 ; 40) à laquelle sont transmis les signaux d'images de tous les capteurs d'images (23).

16. Agencement selon la revendication 15, caractérisé en ce que les dessins (12) portés par le mince revêtement élastique (11) ajusté près du corps et/ou les marques (18) font des contrastes en couleur, et en ce que les capteurs électroniques (23) d'images sont des capteurs d'images sensibles aux couleurs.

17. Agencement selon la revendication 15 ou 16, caractérisé en ce que les capteurs d'images (23) sont disposés dans le dispositif (20) de prise de vues, de façon telle qu'ils saisissent la totalité de la surface du corps ou de la partie (10) du corps à filmer, et en ce que la prise de vues est effectuée en même temps par tous les capteurs d'images (23).
